# EUROPÄISCHE PATENTANMELDUNG

(11) **EP 1 810 678 A1**
(43) Veröffentlichungstag der Anmeldung: **25.07.2007**
(21) Anmeldenummer: 06100578.1
(22) Anmeldetag: 19.01.2006
(51) Int. Cl.: A61K 31/485, A61K 45/06, A61P 25/36, A61P 25/04

(54) **Verwendung einer Kombination von Morphin und Naloxon zur Drogensubstitution**

(71) Anmelder: Hermann, Holger Lars, 8834 Schindellegi (CH)
(72) Erfinder: Hermann, Holger Lars, 8834 Schindellegi (CH)
(74) Vertreter: Hepp, Dieter

(57) **Zusammenfassung**

Die vorliegende Erfindung betrifft die Verwendung von retardiertem Morphin und Naloxon zur Herstellung eines nicht-intravenös darzureichenden Arzneimittels zur Behandlung von Opiatabhängigkeit. Das Präparat zeichnet sich durch ein vorteilhaftes Eigenschaftsprofil aus.

## Beschreibung

Die vorliegende Erfindung betrifft die Verwendung einer Kombination aus Morphin und Naloxon zur Behandlung von Drogenabhängigkeit.

Die Drogensucht ist nach wie vor ein Problem in unserer Gesellschaft. Es ist hinlänglich bekannt, dass die Einnahme bestimmter Substanzen wie beispielsweise Heroin zu einer Abhängigkeit führt. Man unterscheidet hierbei zwischen einer physischen Abhängigkeit und der psychischen Drogenabhängigkeit (Drogensucht). Die physische Drogenabhängigkeit tritt bei einem abrupten Absetzen oder einer signifikanten Verringerung der Menge der eingenommenen Substanz auf. Es kommt zu Entzugserscheinungen wie Erbrechen und Krämpfen des Magen-Darm-Trakts. Hiervon unterscheidet man die eigentliche Drogensucht, die psychische Abhängigkeit, welche als eigene neurologische Erkrankung angesehen wird. Anzeichen von Drogensucht sind die fehlende Kontrolle über die eigene Verwendung der Droge bis hin zur Selbstgefährdung sowie das zwanghafte Verlangen, in den Besitz der Droge zu gelangen.

Drogensucht wird derzeit behandelt, indem man den Patienten substituiert, d.h. von einer gefährlichen auf eine weniger gefährliche Droge umstellt. Aus verschiedenen Gründen findet hierbei aber nicht jede Droge Akzeptanz bei den Patienten. Als Substitutionsmittel wird Methadon, Heroin, Buprenorphin und Morphin eingesetzt. Methadon ist ein synthetisches Opiat mit der Formel

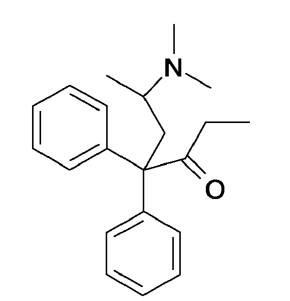

Methadon wird seit den 60er Jahren des 20.Jahrhunderts als Substitutionsmittel eingesetzt, und zwar sowohl in der optisch aktiven L-Form als auch in Form des Racemats. Methadon ist geeignet, die Entzugserscheinungen vor allem bei Heroinpatienten gut zu mildern. Es hat eine längere Wirkdauer als Heroin, kann ohne nennenswerten Wirkungsverlust oral eingenommen werden und ruft keinen "Kick" hervor (d.h. das besonders euphorische Gefühl, dass durch z.B. Heroin ausgelöst wird und zur Drogensucht führt). Um eine missbräuchliche intravenöse Gabe zu verhindern, wird Methadon in Suchtbekämpfungsprogrammen oft als Sirup, gemischt mit Zucker oder Orangensaft verabreicht.

Allerdings ist die Wirkung von Methadon individuell verschieden und daher nicht einfach zu verabreichen. Methadon macht ebenfalls abhängig. Die Entzugserscheinungen bei Methadon sind sogar länger anhaltend als bei Heroin. Methadon führt wie alle Opiate zu Verstopfung, da es lähmend auf die Magen-Darm-Muskulatur wirkt. Zudem scheint Methadon erhebliche Nebenwirkungen zu besitzen, welche denen des vom Markt genommenen Schmerzmittels Vioxx^{®} entsprechen.

Es besteht daher ein Bedarf nach einer anderen und verbesserten Behandlung von Drogensucht.

Gemäß der vorliegenden Erfindung wurde überraschend gefunden, dass eine Kombination aus retardiertem Morphin und Naloxon ein geeignetes Substitutionsmittel zur Behandlung der Drogensucht darstellt. Die vorliegende Erfindung betrifft daher die Verwendung von retardiertem Morphin und Naloxon zur Herstellung eines nicht-intravenös darzureichenden Arzneimittels zur Behandlung von Drogensucht.

Morphin ist ein bekanntes Opiat mit der Formel:

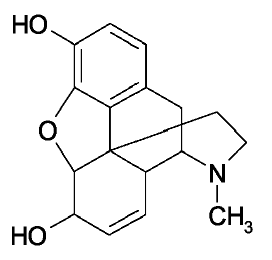

Morphin, umgangssprachlich auch als Morphium bezeichnet, ist ein sehr wirksames Schmerzmittel, das aus den Kapseln des Schlafmohns gewonnen werden kann. Es macht ebenso wie Heroin süchtig, ist aber weniger wirksam.

Gemäß der vorliegenden Erfindung wird das Morphin in retardierter Form verabreicht. Unter einer retardierten Form (oder Retardform) eines Arzneimittels versteht man eine Darreichungsform, bei der der Wirkstoff verlangsamt freigesetzt wird. Mit Hilfe von retardierten Formen kann man den Wirkstoff in kontrollierter Weise im Körper freisetzen und somit möglicherweise gefährliche Konzentrationsspitzen des Wirkstoffs im Blut verhindern. Durch die kontrollierte Abgabe des Wirkstoffs wird eine verlängerte Wirkdauer des Arzneimittels im Patienten erzielt. Der Wirkstoff muss daher weniger oft eingenommen werden.

Gemäß der vorliegenden Erfindung ist eine retardierte Form von Morphin bevorzugt, welche eine nur einmal tägliche Einnahme des Opiats erforderlich macht.

Im Sinn der vorliegenden Erfindung werden die Begriffe "retardierte Form", "Retardform" und "sustained release-Fom" synonym verwendet.

Durch Verwendung von Morphin in retardierter Form muss das Substitutionsmittel weniger häufig eingenommen werden. Aufgrund der länger anhaltenden Wirkung des Morphins wird beim Drogensüchtigen das Verlangen gemildert, sich baldmöglichst neues Rauschmittel besorgen zu müssen. Zudem werden durch die Retardform die Gefahren einer Überdosis gemildert, da der Wirkstoff in kontrollierter Weise unter Vermeidung von Konzentrationsspitzen ins Blut gegeben wird.

Retardierte Formen von Wirkstoffen und insbesondere von Morphin sind dem Fachmann hinlänglich bekannt. Gemäß der vorliegenden Erfindung kann jede retardierte Form von Morphin eingesetzt werden. Bevorzugt ist gemäß der vorliegenden Erfindung eine Formulierung, bei der das Morphin auf einem hydrophilen Polymer adsorbiert und in einer hydrophoben Matrix eingebettet ist. Vorzugsweise handelt es sich bei dem hydrophilen Polymer um ein Cellulose-Polymer und bei der hydrophoben Matrix um ein Wachs. Bei Kontakt mit dem Magensaft bildet das hydrophile Polymer ein Gel, durch welches der Wirkstoff nur unter Verzögerung hindurchtreten kann. Eine andere bevorzugte Retard-Formulierung ist dadurch gekennzeichnet, dass in einer flüssigen Darreichungsform das Morphin in einem Ethylcellulose-Polymer suspendiert und nur verzögert freigesetzt wird. Eine andere gebräüchliche Retardform ist eine mit einer Schutzschicht überzogene Tablette. Die Schutzschicht löst sich im Magensaft nur langsam auf, und der Wirkstoff wird entsprechend verzögert freigesetzt.

Das Morphin kann gemäß der vorliegenden Erfindung in allen üblichen Darreichungsformen verabreicht werden. Bevorzugt sind physiologisch akzeptable wasserlösliche Salze wie das Morphin-Hydrochlorid oder das Morphinsulfat-Pentahydrat.

Gemäß der vorliegenden Erfindung wird das Morphin in Verbindung mit Naloxon gegeben. Naloxon ist ein Opiatantagonist mit der Formel:

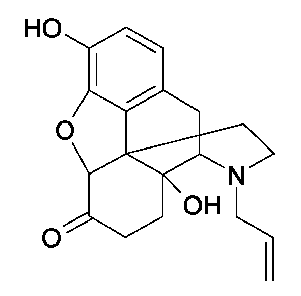

Das Naloxon kann gemäß der vorliegenden Erfindung in allen üblichen Darreichungsformen verabreicht werden. Bevorzugt sind physiologisch akzeptable wasserlösliche Salze wie das Naloxon-Hydrochlorid oder das Naloxon-Hydrochlorid-dihydrat. Das Naloxon liegt im erfindungsgemäßen Präparat nicht in retardierter Form vor.

Naloxon wirkt als Opiatantagonist genau entgegengesetzt zu Opiatantagonisten wie Morphin: Naloxon blockiert die Opiatrezeptoren und vermindert die Wirkung des Morphins. Bei entsprechend hoher Dosierung schaltet Naloxon die Wirkung von Morphin vollständig aus, und es kommt zu Entzugserscheinungen. Dieser Effekt ist bekannt und wird beispielsweise zur Behandlung von Opiat-Vergiftungen durch Gabe von Naloxon ausgenützt.

Bemerkenswerterweise tritt die Opiat-antagonistische Wirkung von Naloxon bei intravenöser Gabe in signifikantem Umfang auf, bei oraler Gabe jedoch kaum. Der Grund hierfür liegt darin, dass Naloxon bei oraler Darreichung einem ausgeprägten First-Pass-Metabolismus unterliegt und daher rasch abgebaut wird. Mit anderen Worten ist die Bioverfügbarkeit von oral verabreichtem Naloxon sehr gering, und die opioide Wirkung des Morphins wird durch das Naloxon kaum beeinträchtigt. Bei intravenöser Einnahme würde das Naloxon jedoch die opioide Wirkung des Morphins erheblich beeinträchtigen. Mit anderen Worten ist der Patient beim erfindungsgemäßen Präparat dazu genötigt, das Mittel nicht intravenös einzunehmen. Die intravenöse Einnahme ist bei Drogensüchtigen aufgrund des schnellen Wirkungseintritts ("Kick") zweifellos die Darreichungsroute der Wahl. Mit dem erfindungsgemäßen Präparat würde die intravenöse Einnahme aber nur zur Frustration führen, da die Wirkung des Morphins durch das intravenöse wirksame Naloxon erheblich reduziert würde.

Die Gefahr einer missbräuchlichen intravenösen Einnahme des erfindungsgemäßen Präparats wird zudem dadurch vermindert, dass das Morphin in retardierter Form vorliegt.

Erfindungsgemäß ist die Menge an Naloxon in dem Präparat so hoch, dass die durch das Morphin verursachte unerwünschte Nebenwirkung der Verstopfung verringert wird.

Es hat sich zudem gezeigt, dass durch die Anwesenheit des Naloxons die sonst auftretende Toleranz des Patienten gegen Morphin verringert wird.

Somit weist das erfindungsgemäße Präparat folgendes überraschendes Profil auf:
- Verringerung der Morphin-verursachten Verstopfung
- Verringerung der Toleranzentwicklung gegen Morphin
- Verhinderung einer missbräuchlichen intravenösen Einnahme (i.v.-abusus)
- Hervorragende Eignung als Substitutionsmittel

Es sind aus dem Stand der Technik bereits Kombinationspräparate aus bestimmten Opioid-Analgetika und Naloxon bekannt. Das Handelsprodukt Talwin^{®}Nx enthält Pentazocin und Naloxon. Das Produkt Valoron^{®}N enthält Tilidin und Naloxon. Es handelt sich hierbei aber um Schmerzpräparate, bei denen das zugesetzte Naloxon eine missbräuchliche Verwendung als Droge verhindern soll. Es wurde bislang nicht erwogen, diese Handelsprodukte als Drogensubstitutionsmittel einzusetzen. Im Stand der Technik wurde vielmehr versucht, die Verwendung der entsprechenden Präparate als Droge zu verunmöglichen.

Es ist weiterhin bereits eine Kombination von Buprenorphin und Naloxon als Drogensubstitutionsmittel bekannt. Allerdings sprechen nur etwa 20 bis 30% aller Drogenabhängigen auf dieses Mittel an.

In dem erfindungsgemäßen Präparat sind 1 bis 2000 mg, vorzugsweise 100 mg bis 1500 mg retardiertes Morphin enthalten. Die Menge an Morphin kann entsprechend den Bedürfnissen des Patienten angepasst werden. Zusätzlich sind 0.1 bis 8 mg Naloxon pro 100 mg retardiertes Morphin, vorzugsweise 1 bis 5 mg Naloxon pro 100 mg retardiertes Morphin oder 1 bis 5 mg pro Tablette enthalten.

Obwohl gemäß der vorliegenden Erfindung vorzugsweise Naloxon als zusätzlicher Wirkstoff eingesetzt wird, sind prinzipiell jedoch alle Opiat-Antagonisten mit fehlender hoher Bioverfügbarkeit bei oraler Gabe einsetzbar. Derartige Verbindungen sind dem Fachmann bekannt. Hinsichtlich Mengenangaben und Darreichungsart gilt für diese Substanzen analog das vorstehend zu Naloxon Ausgeführte.

Das erfindungsgemäße Präparat ist dadurch gekennzeichnet, dass es aus den vorstehenden Gründen nicht intravenös eingenommen werden kann. Das erfindungsgemäße Präparat kann oral (z.B. als Lösung oder Tablette), nasal oder pulmonal (vorzugsweise als Spray) rektal oder als Pflaster appliziert werden. Dem Fachmann sind die entsprechenden Darreichungsformen bekannt. Das erfindungsgemäße Präparat enthält je nach spezifischer Darreichungsform die hierfür üblichen Zusatzstoffe, welche dem Fachmann ebenfalls hinreichend bekannt sind und hier nicht weiter erläutert werden müssen.

## Patentansprüche

1. Verwendung von retardiertem Morphin und mindestens einem Opiat-Antagonisten mit fehlender hoher Bioverfügbarkeit bei oraler Gabe zur Herstellung eines nicht-intravenös darzureichenden Arzneimittels zur Behandlung von Drogensucht.

2. Verwendung nach Anspruch 1, **dadurch gekennzeichnet, dass** der Opiat-Antagonisten mit fehlender hoher Bioverfügbarkeit bei oraler Gabe Naloxon ist.

3. Verwendung nach Anspruch 1 oder 2, **dadurch gekennzeichnet, dass** das Morphin auf einem hydrophilen Polymer adsorbiert und in einer hydrophoben Matrix eingebettet ist.

4. Verwendung nach Anspruch 1 oder 2, **dadurch gekennzeichnet, dass** das Morphin in einem Ethylcellulose-Polymer suspendiert ist.

5. Verwendung nach einem der Ansprüche 1 bis 4, **dadurch gekennzeichnet, dass** das Arzneimittel oral, nasal oder pulmonal, rektal oder als Pflaster appliziert wird.

6. Verwendung nach einem der Ansprüche 1 bis 5, **dadurch gekennzeichnet, dass** es sich um ein einmal täglich einzunehmendes Präparat handelt.

7. Verwendung nach einem der Ansprüche 1 bis 6, **dadurch gekennzeichnet, dass** das Morphin in Form des Morphin-Hydrochlorids oder des Morphinsulfat-Pentahydrats eingesetzt wird.

8. Verwendung nach einem der Ansprüche 1 bis 7, **dadurch gekennzeichnet, dass** das Naloxon in Form des Naloxon-Hydrochlorids oder des Naloxon-Hydrochlorid-dihydrats eingesetzt wird.

9. Verwendung nach einem der Ansprüche 1 bis 8, **dadurch gekennzeichnet, dass** das Arzneimittel 100 mg bis 2000 mg retardiertes Morphin enthält.

10. Verwendung nach einem der Ansprüche 1 bis 9, **dadurch gekennzeichnet, dass** das Arzneimittel 0.1 bis 8 mg Naloxon pro 100 mg retardiertes Morphin enthält.
